# EUROPEAN PATENT SPECIFICATION

(11) **EP 0 729 732 B1**
(45) Date of publication and mention of the grant of the patent: **30.07.2003**
(21) Application number: 95500028.6
(22) Date of filing: 03.03.1995
(51) Int. Cl.: A61F 2/36

(54) **Modular design osseous substitution prosthesis**
Bausteinartige Knochenersatzprothese
Prothèse de substitution osseuse de conception modulaire

(43) Date of publication of application: 04.09.1996
(73) Proprietor: INDUSTRIAS QUIRURGICAS DE LEVANTE, S.A., 46988 Paterna - Valencia (ES)
(72) Inventor: Mas Gomez, Fco. José, F-46988 Paterna, Valencia (ES)
(74) Representative: Koepe, Gerd L., Dipl.-Chem.

(56) References cited:
- EP-A- 0 243 298
- EP-A- 0 382 395
- EP-A- 0 382 429
- EP-A- 0 399 920
- WO-A-94/07438
- DE-A- 4 031 520
- DE-A- 4 320 086
- FR-A- 2 705 558
- US-A- 5 002 578

## Description

The present invention refers to an osseus replacement prosthesis of modular design appropriated for femur proximal zones.

The curative treatment of the primitive malign osseous tumors requires the chirurgical resection "in block" of the whole existing macroscopic desease at the moment of diagnosis.

The existing surgical proceedings to obtain said objective can be framed within two fundamental possibilities: the amputation or the limb preservation technics.

During the recent years, the introduction of new diagnostical means by the image, and the use of the preoperative chemotherapy and radiotherapy have contributed to perform this type of surgery. For this reason, modern technics of limb rescue have been developed. In Spain, various articles have been recently published referring to this pathology type and reconstruction technics thereof.

The primitive osseous tumors, lay mainly on metaphisary level of the long bones, in their more fertile aspect. The reconstruction implies the question of the correction of the articular deficiency, which can only be effected by means of arthrodesis or arthroplasty; although if it is wished to reconstruct functionally the osseus defect, one must make use of an "artificial" arthroplasty (resection prosthesis) or a "natural" arthroplasty (osteoarticular osseous alograft).

With the appearing and development of the bones bands, the osteocartilageous alografts constitute "a priori" the most desireable solution, although there are still problems pending to be resolved, such as the preservation of the articular cartilage or of the articular denervation which leed to a similar situation as that of the neuropathical arthropaty.

The resection prosthesis are the alternative mostly used at present. Some 30 years ago chondrosarcomas and parostal osteosarcomas began to be used in benign tumors with local aggressivity or highly relapsy, such as the tumor with gigant cells, and in malign tumors of low grade.

Recent studies have shown an excellent local control of the disease, preserving the function. Harris, et al made a comparative study among patients who had been amputated and those to whom the limb had been preserved, finding similar functional results, but with a higher emotional acceptability in those to whom a conservative surgery had been submitted.

The recurrence percentage is similar among the amputated and those submitted to a conservative surgery; and also their survival is comparable.

The conservative surgery consists, nowadays, of two surgical proceedings: The first being the tumor resection, which is simply to be evaluated by the local recurrence rates. The second proceeding is the reconstruction, which comes to depend on the resected structures.

There is another application for the prosthesis of osseous resection which is the case of the revisions. At present there are thousands of persons who have been submitted in former years to a substitution of the hip articulation with a prosthesis, these prosthesis as the time goes by became loosened making necessary their substitution. In the case that the bone is in good conditions a prosthesis similar to the former one can be used, however the femur is partially destructed and the best solution is the partial resection of the bone and the substitution with a prosthesis of the type of the object of this invention.

The use of prosthesis for the substitution of the resected zone began with the use of prosthesis properly measured; these prosthesis are being used at present.

The document DE-A 43 20 086 describes a modular shaft hip prosthesis system comprising an anchoring portion, a portion extending the anchoring portion and a hip articulation portion. The three components are put together via conical connecting portions and are secured by means of a securing screw which is inserted into a central bore in all components.

The document DE-A 40 31 520 relates to another modular shaft hip prosthesis system comprising an articulation portion and an anchoring portion which may be put together via fixing elements and then secured by means of a securing screw. In order to establish a variation in length of the prosthesis system, the anchoring portion provides several steps into which the fixing elements may be engaged. Nevertheless, these prosthesis have some inconveniences:
- price
- long execution term
- the intraoperative resection must be adapted to the prosthesis

Therefore nowadays there is a tendancy toward modular prosthesis which are adaptable to the incidences of the surgery action.

To overcome these problems the so called "osseous substitution prosthesis" is proposed.

The present invention relates to an osseous substitution prosthesis of a modular design for the osseous substitution in the femur proximal zone covering up to two thirds of the femur bone, said prosthesis comprising
- a metaphysis component having an axial central bore for receiving a screw;
- one or more than one accumulable selectable diaphysis component(s) having equal or different lengths so as to adapt the prosthesis length to the length of the resection to be effected, said diaphysis component(s) having an axial bore for receiving a screw and having, at either of its two axial ends either a male or a female hexagonal screw nut permitting six different positions and immobilizing the rotation of one component against another;
- an intramedullar stem having a diameter and length depending upon the diameter and length of the medullar channel into which it is to be inserted, said intramedullar stem having at its end proximal to the diaphysis component(s) an axial threaded bore for receiving a screw, a hexagonal screw nut permitting six different positions and immobilizing the rotation of one component against another, and having a lateral tongue allowing a transversal fixation of the stem to the healthy portion of the femural bone by means of cortical screws; and
- a screw adapted in length so as to join said metaphysis component, said one or more than one diaphysis component(s) and said intramedullar stem via their central axial bores and holding the prosthesis screwed in the intramedullar stem.

The invention also relates to an osseous substitution prosthesis of a modular design for the osseous substitution in the femur diaphsis covering up to two thirds of the femur bone, said prosthesis comprising
- one selectable diaphysis component adapting the prosthesis length to the length of the resection to be effected, said diaphysis component having, at either of its two axial ends a female hexagonal screw nut permitting six different positions and immobilizing the rotation of one component against another;
- a distal intramedullar stem having a diameter and length depending upon the diameter and length of the medullar channel into which it is to be inserted, said distal intramedullar stem having at its end proximal to the diaphysis component a male hexagonal screw nut permitting six different positions and immobilizing the rotation of one component against another, and having a lateral tongue allowing a transversal fixation of the stem to the healthy portion of the femural bone by means of cortical screws; and
- a proximal intramedullar stem having a diameter and length depending upon the diameter and length of the medullar channel into which it is to be inserted, said proximal intramedullar stem having at its end proximal to the diaphysis component a hexagonal screw nut permitting six different positions and immobilizing the rotation of one component against another, and having a lateral tongue allowing a transversal fixation of the stem to the healthy portion of the femural bone by means of cortical screws.

In order to better understand the object of the present invention a preferred practical embodiment of the same is represented in the accompanying drawing:

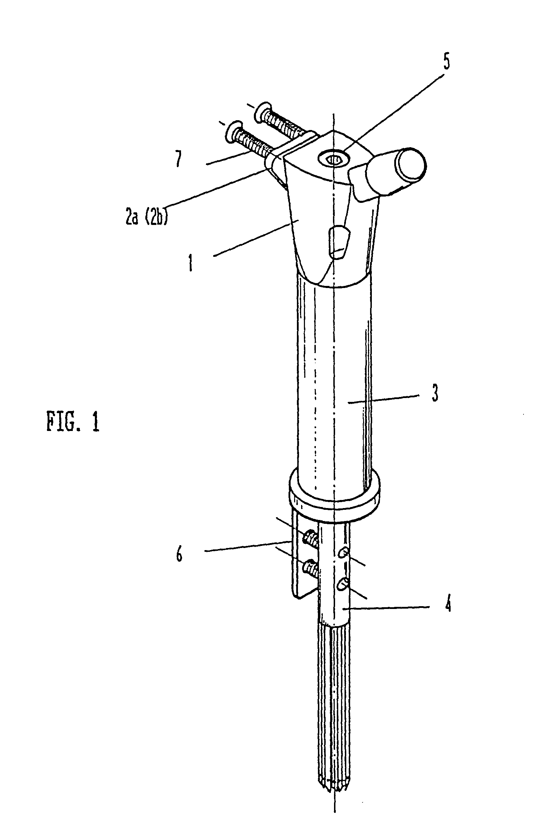

Figure 1 shows a perspective view of the assembled object of the invention.

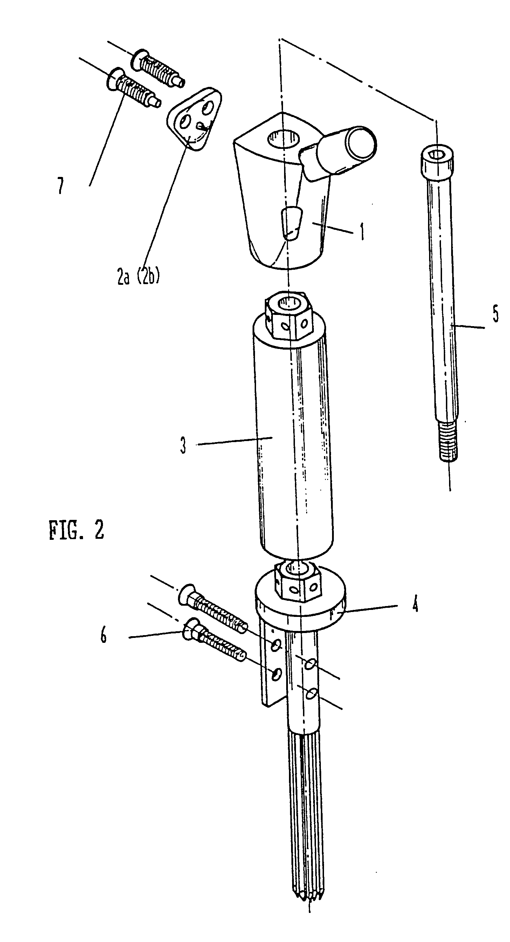

Figure 2 shows a perspective view of each essential element which constitute the object of the invention, in explosion view.
Figure 3 corresponds to an additional mounting with two elements of the invention.

The osseous substitution prosthesis consists of the elements described hereinbelow, basing the design of said elements on the following characteristics:
1) The prosthesis is adequate for osseous substitutions in the femur proximal zones, up to the 2/3 of upper part of the femur.
2) The prosthesis is made of an Ti-6A1-4V alloy which is a material presenting many advantages with respect to others:
   - high elasticity and fracture limit;
   - elasticity module with half the value of steel and of Cr-Co-Mo
   - very good biocompatibility
   - stress limit higher than that of steel
   - density half the value of steel and of Cr-Co-Mo.
3) The prosthesis is designed such that its utilization is flexible and that during surgical intervention, any variation over what is scheduled is made possible. For that reason they are provided with a sole metaphysis component 1 which is common for every intervention. Depending on the length of the resection to be effected, one or more than one diaphysis components 3 is/are used, as these may be accumulated. The rate of increase from one size to a next one is of 20 mm, with which the maximum error that might be committed is of 20 mm. This error can be compensated by reducing the bone length a little more than what is strictly necessary and/or utilizing bar heads with different necks 1 and/or cups with various profiles with which the cited defect is corrected. There are 6 intramedullar stems (2 lengths and 3 diameters) which are chosen depending on the diameter of the patient's medullar canal and on the quality of the bone. Normally, long intramedullar stems are used in resection prosthesis and short intramedullar stems in tumorations.
   In order to join the metaphysis component 1, one or more than one diaphysis components 3 and the intramedullar stem a screw is used which holds the prosthesis being screwed in the intramedullar stem. There are 11 union screws 5 which permit to utilize diaphysis components 3 from 0 to 200 mm.
4) The metaphysis component 1 has a medial opening to connect the ligaments and in the rear zone permits the annexation of the trochanter ligaments. This is achieved by screwing the fixation plate with the trochanter fixation screws 7. In the case where the trochanter can be saved, the trochanter fixation plate trochanterical plate 2 is used, while if it cannot be saved, the ligaments are direclty connected to the ligament fixation plate.
5) The intramedullar stem is introduced in the healthy bone and for improving its fixation cortical screws 6 are used which transversally fix the lateral tongue of the stem to the bone.
6) There is the possibility to make resections of the femur diaphysis, preserving the hip articulation. This solution is possible, in the case that the tumor is located in the diaphysis and does not affect the hip nor the knee. The mounting to undertake is in this case different and consists of joining the proximal intramedullar stem 9 with a diaphysis component 3 and with a distal intramedullar stem 4. The joining of these elements is obtained with transversal screws not represented in the accompanying drawings. There are three sizes of proximal intramedullar stems 9 with the same length but with different diameters.
7) The fitting of the different pieces with each other is obtained by means of male and female hexagonal screw nuts which permit 6 different positions and that immobilize the rotation of one element against the other.
8) The prosthesis have a porous coating in the following zones:
   - total front surface of the metaphysis component 1 and diaphysis components 3 in order to situate the bone graft in this zone and in order to achieve the bone osteointegration with the implant.
   - proximal one third portion of the intramedullar stem in order to achieve a good fixation of the implant to the bone.
9) The metaphysis component 1 is unique and its size is reduced in order that the minimum resection to undertake is small.

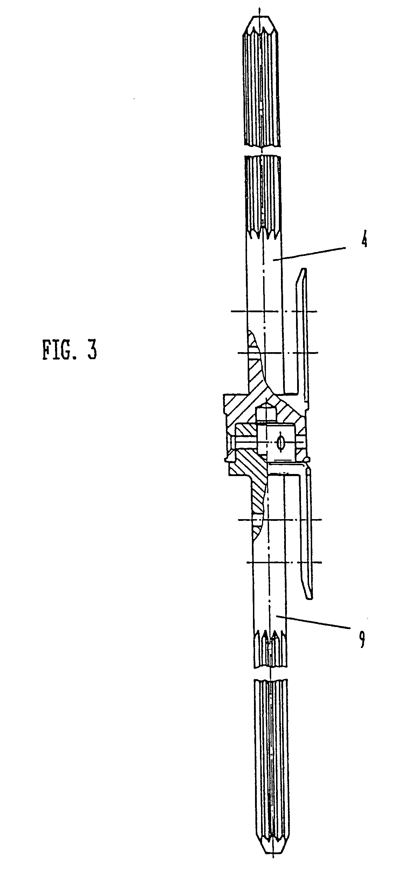

Resuming and as it has shown hereinabove, the prosthesis has a modular design which is adjusted to the surgeon's needs and that it joined to its high stability, its light weight. All its elements are combinable and enable to solve the majority of the cases which are presented in the case of massive osseous substitutions of the proximal femur. A combination case may be the one shown in figure 3 where a prosthesis with the utilization of both, the proximal 9 and the distal 4 intramedullar stems, is shown.

Once sufficiently described the nature of the present invention, as well as the form to put it into practice, it only remains to be added that in the whole invention and the parts of which it is compose of, it is possible to introduce changes of form, materials and disposition, provided that said alterations do not vary substantially the characteristics of the invention which are claimed as follows.

## Claims

1. Osseous substitution prosthesis of a modular design for the osseous substitution in the femur proximal zone covering up to two thirds of the femur bone, said prosthesis comprising
- a metaphysis component (1) having an axial central bore for receiving a screw;
- one or more than one accumulable selectable diaphysis component(s) (3) having equal or different lengths so as to adapt the prosthesis length to the length of the resection to be effected, said diaphysis component(s) (3) having an axial bore for receiving a screw and having, at either of its two axial ends either a male or a female hexagonal screw nut permitting six different positions and immobilizing the rotation of one component against another;
- an intramedullar stem (4) having a diameter and length depending upon the diameter and length of the medullar channel into which it is to be inserted, said intramedullar stem (4) having at its end proximal to the diaphysis component(s) (3) an axial threaded bore for receiving a screw, a hexagonal screw nut permitting six different positions and immobilizing the rotation of one component against another, and having a lateral tongue allowing a transversal fixation of the stem to the healthy portion of the femural bone by means of cortical screws (6); and
- a screw (5) adapted in length so as to join said metaphysis component (1), said one or more than one diaphysis component(s) (3) and said intramedullar stem (4) via their central axial bores and holding the prosthesis screwed in the intramedullar stem (4).

2. Osseous substitution prosthesis of a modular design for the osseous substitution in the femur diaphysis covering up to two thirds of the femur bone, said prosthesis comprising
- one selectable diaphysis component (3) adapting the prosthesis length to the length of the resection to be effected, said diaphysis component (3) having, at either of its two axial ends a female hexagonal screw nut permitting six different positions and immobilizing the rotation of one component against another;
- a distal intramedullar stem (4) having a diameter and length depending upon the diameter and length of the medullar channel into which it is to be inserted, said distal intramedullar stem (4) having at its end proximal to the diaphysis component (3) a male hexagonal screw nut permitting six different positions and immobilizing the rotation of one component against another, and having a lateral tongue allowing a transversal fixation of the stem to the healthy portion of the femural bone by means of cortical screws (6); and
- a proximal intramedullar stem (9) having a diameter and length depending upon the diameter and length of the medullar channel into which it is to be inserted, said proximal intramedullar stem (9) having at its end proximal to the diaphysis component (3) a hexagonal screw nut permitting six different positions and immobilizing the rotation of one component against another, and having a lateral tongue allowing a transversal fixation of the stem to the healthy portion of the femural bone by means of cortical screws (6).

3. The osseous substitution prosthesis according to claim 1, wherein the metaphysis component (1) has a medial opening for connecting ligaments and/or has a rear zone which permits the annexation of trochanter ligaments by means of a fixation plate and trochanter fixation screws (7).

4. The osseous substitution prosthesis according to any of claims 1 to 3, wherein the diaphysis component (3) comprises one or more than one modular element(s) which may be combined to each other in order to achieve the required length adapted to the length of the resection to be effected, allowing for a maximum error in proportion to the length of the shortest possible diaphysis component (3), the length differences of the possible diaphysis components (3) being in intervals of 20 mm from one diaphysis component length to the next larger/smaller one.

5. The osseous substitution prosthesis according to any of the claims 1 to 4, wherein a porous coating is provided on the external surfaces of the metaphysis component (1) and of the diaphysis component (3).

6. The osseous substitution prosthesis of claim 5, wherein a porous coating is also provided on the proximal portion of the intramedullar stem (4, 9).

7. The osseous substitution prosthesis according to claim 2, wherein the proximal intramedullar stem (9) may have different diameters but has always the same length.

## Patentansprüche

1. Knochenersatz-Prothese mit modularem Design zum Knochenersatz in der proximalen Zone des Femur, die bis zu zwei Drittel des Femur-Knochens erfaßt, wobei die Prothese umfasst:
- eine Metaphyse-Komponente (1) mit einer axialen zentralen Bohrung für die Aufnahme einer Schraube;
- eine oder mehr als eine addierbar verwendbare, wählbare Diaphyse-Komponente(n) (3), die gleiche oder verschiedene Längen aufweisen, so dass die Prothesenlänge der Länge der zu bewirkenden Resektion angepaßt werden kann, wobei die Diaphyse-Komponente(n) (3) eine axiale Bohrung zur Aufnahme einer Schraube aufweist/aufweisen und an jedem seiner/ihrer axialen Enden entweder eine männliche oder eine weibliche hexagonale Schraubenmutter aufweist/aufweisen, die sechs verschiedene Positionen zuläßt und die Rotation einer Komponente gegen eine andere immobilisiert;
- einen intramedullären Stab (4), der einen Durchmesser und eine Länge aufweist, die von dem Durchmesser und der Länge des Medullar-Kanals abhängen, in den er eingeschoben werden soll, wobei der intramedulläre Stab (4) an seinem zu der/den Diaphyse-Komponente(n) (3) proximal gelegenen Ende eine axiale Gewindebohrung zur Aufnahme einer Schraube aufweist, eine hexagonale Schraubenmutter aufweist, die sechs verschiedene Positionen zuläßt und die Rotation einer Komponente gegen eine andere immobilisiert und die eine seitliche Zunge aufweist, die eine transversale Fixierung des Stabs an dem gesunden Abschnitt des Femur-Knochens mittels kortikaler Schrauben (6) ermöglicht; und
- eine Schraube (5), die hinsichtlich ihrer Länge so angepaßt ist, dass sie die Metaphyse-Komponente (1), die eine oder mehr als eine Diaphyse-Komponente(n) (3) und den intramedullären Stab (4) über ihre zentralen axialen Bohrungen verbindet und die Prothese in dem intramedullären Stab (4) festgeschraubt hält.

2. Knochenersatz-Prothese mit modularem Design zum Knochenersatz in der Femur-Diaphyse, die bis zu zwei Dritteln des Femur-Knochens erfaßt, wobei die Prothese umfaßt:
- eine selektierbare Diaphyse-Komponente (3), die die Prothesenlänge der Länge der zu bewirkenden Resektion anpaßt, wobei die Diaphyse-Komponente (3) an jedem ihrer beiden axialen Enden eine weibliche hexagonale Schraubenmutter aufweist, die sechs verschiedene Positionen erlaubt und die Rotation einer Komponente gegen eine andere immobilisiert;
- einen distalen intramedullären Stab (4), der einen Durchmesser und eine Länge aufweist, die abhängig sind von dem Durchmesser und der Länge des Medullar-Kanals, in den er eingesetzt werden soll, wobei der distale intramedulläre Stab (4) an seinem der Diaphyse-Komponente (3) proximalen Ende eine männliche hexagonale Schraubenmutter aufweist, die sechs verschiedene Positionen erlaubt und die Rotation einer Komponente gegen eine andere immobilisiert, und der eine seitliche Zunge aufweist, die eine transversale Fixierung des Stabs an dem gesunden Abschnitt des Femur-Knochens mittels kortikaler Schrauben (6) erlaubt; und
- einen proximalen intramedullären Stab (9), der einen Durchmesser und eine Länge aufweist, die von dem Durchmesser und der Länge des Medullar-Kanals abhängen, in den er eingesetzt werden soll, wobei der proximale intramedulläre Stab (9) an seinem zu der Diaphyse-Komponente (3) proximalen Ende eine hexagonale Schraubenmutter aufweist, die sechs verschiedene Positionen erlaubt und die Rotation einer Komponente gegen eine andere immobilisiert und der eine seitliche Zunge aufweist, die eine transversale Fixierung des Stabs an dem gesunden Abschnitt des Femur-Knochens mittels kortikaler Schrauben (6) erlaubt.

3. Knochenersatz-Prothese nach Anspruch 1 worin die Metaphyse-Komponente (1) eine mediale Öffnung zum Anbinden von Ligamenten aufweist, und/oder einen hinteren Bereich aufweist, der den Anschluß von Trochanter-Ligamenten mittels einer Fixierungsplatte und Trochanter-Fixierungsschrauben (7) erlaubt.

4. Knochenersatz-Prothese nach einem der Ansprüche 1 bis 3, worin die Diaphyse-Komponente (3) ein oder mehr als ein modulare(s) Element(e) umfaßt, das/die miteinander kombiniert werden können, um die erforderliche Länge zu erzielen, die an die Länge der zu bewirkenden Resektion angepaßt ist, was einen maximalen Fehler im Verhältnis zur Länge der kürzest-möglichen Diaphyse-Komponente (3) ermöglicht, wobei die Längen-Unterschiede der möglichen Diaphyse-Komponenten (3) in Intervallen von 20 mm von der Länge einer Diaphyse-Komponente zur nächstgrößeren bzw. nächst-kleineren liegen.

5. Knochenersatz-Prothese nach einem der Ansprüche 1 bis 4 worin ein poröser Überzug auf den Außenflächen der Metaphyse-Komponente (1) und der Diaphyse-Komponente (3) vorgesehen ist.

6. Knochenersatz-Prothese nach Anspruch 5, worin ein poröser Überzug auch auf dem proximalen Abschnitt des intramedullären Stabs (4, 9) vorgesehen ist.

7. Knochenersatz-Prothese nach Anspruch 2, worin der proximale intramedulläre Stab (9) verschiedene Durchmesser aufweisen kann, jedoch immer dieselbe Länge hat.

## Revendications

1. Prothèse de substitution osseuse de conception modulaire pour la substitution osseuse dans une zone de fémur proximal recouvrant jusqu'à deux tiers de l'os du fémur, ladite prothèse comprenant :
- un composant de métaphyse (1) ayant un alésage central axial pour recevoir une vis ;
- un ou plus d'un composant de diaphyse (3) qui peuvent être sélectionnés et cumulés ayant des longueurs égales ou différentes afin d'adapter la longueur de la prothèse à la longueur de la résection à effectuer, ledit (lesdits) composant(s) de diaphyse (3) ayant un alésage axial pour recevoir une vis et ayant à ses (leurs) deux extrémités axiales un écrou de vis hexagonal soit mâle soit femelle permettant six positions différentes et immobilisant la rotation d'un composant par rapport à un autre;
- une tige intramédullaire (4) ayant un diamètre et une longueur en fonction du diamètre et de la longueur du canal médullaire dans lequel elle doit être insérée, ladite tige intramédullaire (4) ayant à son extrémité proximale à (aux) composant(s) de diaphyse (3) un alésage axial fileté pour recevoir une vis, un écrou de vis hexagonal permettant six positions différentes et immobilisant la rotation d'un composant par rapport à un autre, et ayant une langue latérale permettant une fixation transversale de la tige à la partie saine de l'os fémoral au moyen de vis corticales (6) ; et
- une vis (5) adaptée en longueur afin de joindre ledit composant de métaphyse (1), ledit un ou plus d'un composant de diaphyse (3) et ladite tige intramédullaire (4) par leurs alésages axiaux centraux, et maintenant ladite prothèse visée dans la tige intramédullaire (4).

2. Prothèse de substitution osseuse de conception modulaire pour la substitution osseuse dans la diaphyse d'un fémur recouvrant jusqu'à deux tiers de l'os du fémur, ladite prothèse comprenant :
- un composant de diaphyse (3) qui peut être sélectionné et qui adapte la longueur de la prothèse à la longueur de la résection à être effectuée, ledit composant de diaphyse (3) ayant à chacune de ses deux extrémités axiales un écrou de vis femelle hexagonal permettant six positions différentes et immobilisant la rotation d'un composant par rapport à un autre ;
- une tige intramédullaire distale (4) ayant un diamètre et une longueur en fonction du diamètre et de la longueur du canal médullaire dans lequel elle doit être insérée, ladite tige intramédullaire distale (4) ayant à son extrémité proximale au composant de diaphyse (3) un écrou de vis mâle hexagonal permettant six positions différentes et immobilisant la rotation d'un composant par rapport à un autre, et ayant une langue latérale permettant une fixation transversale de la tige à la partie saine de l'os fémoral au moyen de vis corticales (6) ; et
- une tige intramédullaire proximale (9) ayant un diamètre et une longueur en fonction du diamètre et de la longueur du canal médullaire dans lequel elle doit être insérée, ladite tige intramédullaire proximale (9) ayant à son extrémité proximale au composant de diaphyse (3) un écrou de vis hexagonal permettant six positions différentes et immobilisant la rotation d'un composant par rapport à un autre, et ayant une langue latérale permettant une fixation transversale de la tige à la partie saine de l'os fémoral au moyen de vis corticales (6).

3. La prothèse de substitution osseuse selon la revendication 1, dans laquelle le composant de métaphyse (1) a une ouverture médiane pour connecter des ligaments et/ou a une zone arrière qui permet l'annexion de ligaments du trochanter au moyen d'une plaque de fixation et de vis de fixation du trochanter (7).

4. La prothèse de substitution osseuse selon l'une quelconque des revendications 1 à 3, dans laquelle le composant de diaphyse (3) comprend un ou plus d'un élément modulaire qui peuvent être combinés entre eux afin d'obtenir la longueur nécessaire adaptée à la longueur de la résection à être effectuée, tenant compte d'une erreur maximale en rapport à la longueur du composant de diaphyse (3) le plus petit possible, les différences de longueur des composants de diaphyse possibles (3) présentant des intervalles de 20 mm en partant d'une longueur de composant de diaphyse vers la suivante plus petite/plus grande.

5. La prothèse de substitution osseuse selon l'une quelconque des revendications 1 à 4, dans laquelle un revêtement poreux est prévu sur les surfaces externes du composant de métaphyse (1) et du composant de diaphyse (3).

6. La prothèse de substitution osseuse selon la revendication 5, dans laquelle un revêtement poreux est aussi prévu sur la partie proximale de la tige intramédullaire (4, 9).

7. La prothèse de substitution osseuse selon la revendication 2, dans laquelle la tige intramédullaire proximale (9) peut avoir des diamètres différents, mais a toujours la même longueur.
